# EUROPEAN PATENT APPLICATION

(11) **EP 4 744 687 A1**
(43) Date of publication of application: **20.05.2026**
(21) Application number: 24383249.0
(22) Date of filing: 18.11.2024
(51) Int. Cl.: A61L 31/04

(54) **A COMBINATION, MEDICAL KIT AND MEDICAL DEVICE FOR TREATING AND/OR PREVENTING TISSUE LEAKAGE**

(71) Applicant: Aesculap AG, 78532 Tuttlingen (DE); Viscofan España, S.L.U., 31192 Tajonar (ES); Fundació Institut de Investigació en Ciències de la Salut Germans Trias i Pujol, 08916 Badalona (Barcelona) (ES)
(72) Inventor: WEIS, Christine, Sant Cugat del Vallés (ES); IZCO ZARATIEGUI, Jesús María, TAJONAR (ES); TURÓN, Pau, RUBÍ (ES); DIAZ, Elena, RUBÍ (ES); JULIÁN IBÁNEZ, Juan Francisco, BADALONA (ES); GENÉ KRABEC, Clara, BADALONA (ES); CREMADES PÉREZ, Manel, BADALONA (ES); ZÚÑIGA ARRARÁS, Teresa, TAJONAR (ES); GUEMBE LAPUENTE, Amaia, TAJONAR (ES); RECALDE IRURZUN, José Ignacio, TAJONAR (ES)
(74) Representative: Patentanwälte Ruff, Wilhelm, Beier, Dauster & Partner mbB

(57) **Abstract**

The invention relates to a combination for treating tissue leakage, comprising
- a collagen and/or gelatin material and
- a cyanoacrylate component.

Further, the invention relates to a medical kit and medical device for treating and/or preventing tissue leakage.

## Description

### FIELD OF INVENTION

The present invention refers to a combination, a medical kit and a medical device for treating and/or preventing tissue leakage.

### BACKGROUND OF THE INVENTION

It is known to apply stapler lines to prevent tissue leakage. However, even if correctly placed, microholes may occur enhancing the risk of leakage, which can have devastating consequences for animals and human patients.

### OBJECT AND SOLUTION

In view of the foregoing, an object underlying the present invention is therefore to make available a combination, a medical kit and a medical device useful for treating and/or preventing tissue leakage, which in particular addresses disadvantages or risk as described above.

This object is accomplished by a combination according to independent claim 1, a medical kit according to claim 14 and a medical device according to claim 15. Preferred embodiments of the invention are defined in the dependent claims and the description. The subject-matter and wording, respectively, of all claims is hereby incorporated into the description by explicit reference.

According to a first aspect, the present invention refers to a combination, in particular medical, preferably surgical, combination, in particular for treating and/or preventing tissue leakage.

The combination comprises the following:
- a collagen and/or gelatine material and
- a cyanoacrylate component.

The term "tissue leakage" as used according to the present invention refers to any trauma of tissue, in particular human and/or animal tissue, resulting in the exit of body fluids such as intestinal liquids, bile, feces and blood.

The tissue leakage according to the present invention may be selected from the group consisting of skin leakage, liver leakage, spleen leakage, pancreas leakage, kidney leakage, lung leakage, adrenal gland leakage, stomach leakage and intestines leakage such as small intestines leakage.

The term "collagen and/or gelatine material" as used according to the present invention refers to a material comprising or consisting of collagen and/or gelatine.

The term "gelatine" as used according to the present invention refers to denatured or hydrolyzed collagen. Thus, the term "gelatine" as used according to the invention may in particular refer to collagen hydrolysate, i.e. a collection of peptides and/or proteins, in particular produced by partial hydrolysis of collagen. During hydrolysis, some of the bonds between and within component proteins are broken. The chemical composition of gelatine may be, in many aspects, closely similar to that of its parent collagen. The gelatine may have a molecular weight of 100 kilo Dalton to 500 kilo Dalton, in particular 150 kilo Dalton to 250 kilo Dalton.

The present invention is based on the surprising finding that a combination comprising collagen and/or gelatine material and a cyanoacrylate component may be used for treating and/or preventing tissue leakages, in particular in human beings or animals. In that regard, it was especially surprising that a collagen and/or gelatine material equipped with the cyanoacrylate component exhibited flexibility, impermeability to body fluids such as blood and tissue adherence. Further, collagen is part of healthy tissues. In particular, depending on the type of collagen, the collagen may have or mimic vessel-like structures. Thus, the collagen and/or gelatine material may advantageously foster tissue healing processes such as granulation. Further, the cyanoacrylate component and the collagen and/or gelatine are biodegradable/bioabsorbable materials and may therefore be degraded/absorbed in vivo, in particular when a mechanical support of the collagen and/or gelatine material is no longer needed.

In an embodiment of the invention, the collagen and/or gelatine material is in the form of a two-dimensional surface structure, in particular flat or planar two-dimensional surface structure.

In a further embodiment of the invention, the collagen and/or gelatine material is in the form of a patch, fabric, non-woven, membrane, sponge or a combination of at least two of the aforementioned forms. In particular, the collagen and/or gelatine material may be in the form of a membrane and/or sponge. The size of the aforementioned forms of the collagen and/or gelatine material are especially adjustable to the treating site to be addressed.

Further, the collagen and/or gelatin material may have a layered structure, in particular mono-layered or multi-layered structure, for example two-layered, three-layered, four-layered or five-layered structure.

Further, the collagen and/or gelatine material may be in a lyophilized form, i.e. may have lyophilized structure.

Further, the collagen material may have a cross-linked structure, in particular physically and/or chemically cross-linked structure. Thus, the tensile strength of the collagen and/or gelatine material may be advantageously increased. In particular, the collagen and/or gelatine material may be cross-linked via cross-linking agent, in particular selected from the group consisting of diamine, carbodiimide, diisocyanate, dicarboxylic acids, dialdehydes, polyaldehydes and mixtures of at least two of the aforementioned cross-linking agents. The polyaldehydes may preferably be polysaccharides carrying aldehyde groups (polyaldehydic polysaccharides). The polyaldehydic polysaccharides can in particular have a degree of oxidation of 10 % to 50 %, preferably 10 % to 30 %, for example 25 %. The carbodiimide may be EDC (1-ethyl-3-(3-dimethylaminopropyl)carbodiimide hydrochloride), for example.

In a further embodiment of the invention, the collagen and/or gelatine material is impermeable to cells.

The term "cells" as used according to present invention typically means cells of the body, in particular cells of the human and/or animal body.

In a further embodiment of the invention, the collagen and/or gelatine material is permeable to cells.

Further, the collagen and/or gelatine material may have a mean pore diameter, in particular determined by using a micrometer, in particular a digital micrometer, of ≥ 6 µm, in particular 6 µm to 480 µm, preferably 10 µm to 65 µm.

Further, the collagen and/or gelatin material may have a thickness, in particular layer thickness, of 0.5 mm to 10 mm.

Further, the collagen and/or gelatine material may have a basis weight, i.e. weight per unit area, of 16 g/m² to 150 g/m², preferably 20 g/m² to 45 g/m².

Further, the collagen and/or gelatine material may be in the form of particles, in particular in the form of a powder.

Further, the collagen and/or gelatine material may have a tensile strength of 0.3 N (Newton) to 32 N (Newton), preferably 1 N (Newton) to 10 N (Newton).

Further, the collagen and/or gelatine material may be in the form of a gel, in particular hydrogel. Preferably, the gel is in the form of an injectable gel, in particular viscoelastic injectable gel.

Further, the collagen and/or gelatine material may be in an extruded form. Advantageously, extrusion allows adjustment of layer thickness of the collagen and/or gelatine material. Principally, different collagen manufacturing processes may be used for manufacturing the collagen and/or gelatine material.

In a further embodiment of the invention, the collagen and/or gelatine material comprises or consists of collagen selected from the group consisting of collagen type I, collagen type II, collagen type III, collagen type IV, collagen type V, recombinant collagen and mixtures of at least two of the afore-mentioned collagen types.

Collagen type I is found in skin, tendon, vasculature, organs and bone. With respect to bone, collagen type I forms the main component of the organic part of bone.

Collagen type II is found in cartilage. Collagen type II forms the main collagenous component of cartilage.

Collagen type III is found in reticular fibers. Collagen type III forms the main component of reticular fibers. Commonly, collagen type III is found alongside collagen type I.

Collagen type IV is part of basal lamina and the epithelium-secreted layer of the basement membrane.

Collagen type V is found on cell surfaces, in hair and in placenta.

The recombinant collagen may be synthesized by biosynthetic methods, in particular by the aid of genetically modified microorganisms or in cell cultures. In particular, the recombinant collagen may be capable of forming tissue specific structures, including fibrils, networks, membrane structures etc.

In a further embodiment of the invention, the collagen and/or gelatine material comprises a biological tissue and/or an extracellular matrix (ECM), in particular in a processed form. Preferably, the biological tissue and/or extracellular matrix (ECM) is selected from the group consisting of pericardium, pericardium fibrosum, pericardium serosum, epicardium, squamous epithelium, tunica serosa, muscle such as myocardium, peritoneum, small intestine, small intestinal submucosa, gastric submucosa, urinary bladder submucosa, uterine submucosa, dermis, serosa dermis, and mixtures of at least two of the afore-mentioned biological tissues and extracellular matrices, respectively.

In a further embodiment of the invention, the collagen and/or gelatine material is of xenogenic, in particular bovine, porcine, equine, ovine, or marine origin.

In a further embodiment of the invention, the collagen and/or gelatine material comprises a membranous structure and/or a spongy structure. More preferably, the collagen and/or gelatine material comprises a membranous structure and a spongy structure. In this embodiment, the membranous structure is preferably impermeable to cells and the spongy structure is preferably permeable to cells. Further, the spongy structure may have in particular columnar pores, in particular extending along a thickness direction of the spongy structure. Further, the membranous structure may have a thickness, in particular layer thickness, of 0.1 mm to 5 mm, in particular 0.5 mm to 2 mm , while the spongy structure may preferably have a thickness, in particular layer thickness, of 1 mm to 10 mm, in particular 2 mm to 5 mm. Further, the membranous structure may have weight per unit area, of 50 g/m² to 180 g/m², in particular 80 g/m² to 110 g/m², while the spongy structure may preferably have a weight per unit area, of 30 g/m² to 300 g/m², in particular 80 g/m² to 120 g/m². Preferably, the membranous component is pericardium, while the spongy component is preferably collagen, in particular selected from the group consisting of collagen type I, collagen type II, collagen type III, collagen type IV, collagen type V, recombinant collagen and mixtures of at least two of the afore-mentioned collagen types.

In a further embodiment of the invention, the collagen and/or gelatine material is in the form of an implant, preferably surgical implant.

Specifically, the collagen and/or gelatine material may be in the form of a collagen membrane, i.e. a membrane comprising or consisting of collagen, preferably skin collagen (so-called skin collagen membrane), in particular bovine skin collagen (so-called bovine skin collagen membrane). In particular, the collagen and/or gelatine material may be in the form of an extruded bovine skin collagen membrane.

Further, the collagen and/or gelatine material may be in the form of a pericardium and/or collagen membrane, i.e. a membrane comprising or consisting of pericardium and/or collagen in particular bovine pericardium and/or bovine collagen membrane (so-called bovine pericardium and/or collagen membrane), preferably lyophilized pericardium and/or collagen (so-called lyophilized bovine pericardium and/or collagen membrane). Useful membranes are, for example, commercialized under Lyograft or Lyoplant.

In a further embodiment of the invention, the cyanoacrylate component includes cyanoacrylate monomers represented by the formula

CH₂=CH(CN)-COOR (formula I)

wherein R is an alkyl, alkoxyalkyl, cycloalkyl, alkenyl, aralkyl, aryl, allyl or haloalkyl group.

In particular, the cyanoacrylate component may include cyanoacrylate monomers selected from the group consisting of alkyl 2-cyanoacrylate monomers, alkoxyalkyl 2-cyanoacrlate monomers, multifunctional cyanoacrylate monomers and mixtures of at least two of the afore-mentioned cyanoacrylate monomers.

In a further embodiment of the invention, the cyanoacrylate component includes alkyl 2-cyanoacrylate monomers. The alkyl 2-cyanoacrylate monomers are in particular selected from the group consisting of methyl 2-cyanoacrylate monomers, ethyl 2-cyanoacrylate monomers, n-propyl 2-cyanoacrylate monomers, isopropyl 2-cyanoacrylate monomers, n-butyl 2-cyanoacrylate monomers, isobutyl 2-cyanoacrylate monomers such as, for example, 1-butyl 2-cyanoacrylate monomers and/or 2-butyl 2-cyanoacrylate monomers, n-pentyl 2-cyanoacrylate monomers, isopentyl 2-cyanoacrylate monomers such as, for example, 1-pentyl 2-cyanoacrylate monomers, 2-pentyl 2-cyanoacrylate monomers and/or 3-pentyl 2-cyanoacrylate monomers, cyclopentyl 2-cyanoacrylate monomers, n-hexyl 2-cyanoacrylate monomers, isohexyl 2-cyanoacrylate monomers such as, for example, 1-hexyl 2-cyanoacrylate monomers, 2-hexyl 2-cyanoacrylate monomers, 3-hexyl 2-cyanoacrylate monomers and/or 4-hexyl 2-cyanoacrylate monomers, cyclohexyl 2-cyanoacrylate monomers, n-heptyl 2-cyanoacrylate monomers, isoheptyl 2-cyanoacrylate monomers such as, for example, 1-heptyl 2-cyanoacrylate monomers, 2-heptyl 2-cyanoacrylate monomers, 3-heptyl 2-cyanoacrylate monomers and/or 4-heptyl 2-cyanoacrylate monomers, n-octyl 2-cyanoacrylate monomers, isooctyl 2-cyanoacrylate monomers such as, for example, 1-octyl 2-cyanoacrylate monomers, 2-octyl 2-cyanoacrylate monomers, 3-octyl 2-cyanoacrylate monomers and/or 4-octyl 2-cyanoacrylate monomers, n-nonyl 2-cyanoacrylate monomers, isononyl 2-cyanoacrylate monomers, n-decyl 2-cyanoacrylate monomers, isodecyl 2-cyanoacrylate monomers, n-undecyl 2-cyanoacrylate monomers, isoundecyl 2-cyanoacrylate monomers, n-dodecyl 2-cyanoacrylate monomers, isododecyl 2-cyanoacrylate monomers and mixtures of at least two of the afore-mentioned alkyl 2-cyanoacrylate monomers.

Preferably, the cyanoacrylate component includes n-butyl 2-cyanoacrylate monomers and/or n-octyl 2-cyanoacrylate monomers, more preferably n-butyl 2-cyanoacrylate monomers.

Further, the collagen and/or gelatine material may have a coating. The coating may be in particular formed on two opposite surfaces, in particular main surfaces, of the collagen and/or gelatine material. The coating may comprise a polymer and/or therapeutic agent, in particular as detailed in the following paragraphs.

Further, the combination may further comprise a polymer. The polymer may be part of a coating of the collagen and/or gelatine material and/or dispersed in the collagen and/or gelatine material. Accordingly, the collagen and/or gelatine material may be coated with a polymer. Alternatively, for manufacture of the collagen and/or gelatine material, collagen and/or gelatine may be mixed with a polymer.

In particular, the polymer may be selected from the group consisting of polyvinyl alcohol, polyglycolide or polyglycolic acid, polylactide or polylactic acid, polydioxanone, polyhydroxybutyrate or polyhydroxybutyric acid, poly-3-hydroxybutyrate or poly-3-hydroxybutyric acid, poly-4-hydroxybutyrate or poly-4-hydroxybutyric acid, polytrimethylene carbonate, poly-ε-caprolactone, polyethylene glycol, elastin, reticulin, fibronectin, laminin, fibrin, albumin, starch, amylose, amylopectin, dextran, dextrin, cellulose, cellulose derivatives such as methylcellulose, hydroxymethylcellulose, hydroxyethylcellulose, hydroxypropylcellulose, carboxymethylcellulose, chitin, chitosan, hyaluronic acid, dextran sulfate, heparin, heparan sulfate, chondroitin sulfate, dermatan sulfate, polyethylene glycol and mixtures of at least two of the aforementioned polymers.

Further, the combination may further comprise a therapeutic agent. The therapeutic agent may be part of a coating of the collagen and/or gelatine material and/or dispersed in the collagen and/or gelatine material. Accordingly, the collagen and/or gelatine material may be coated with the therapeutic agent. Alternatively, for manufacture of the collagen and/or gelatine material, collagen and/or gelatine may be mixed with the therapeutic agent.

In particular, the therapeutic agent may be selected from the group consisting of antimicrobial, in particular antibiotic, agents, disinfecting agents, anti-inflammatory agents, wound healing promoting agents, cellular growth agents, morphogenetic agents, cytokines, peptides, proteins, extracellular components, cellular differentiating agents, cellular adhesion agents, cellular recruiting agents, anesthetic agents, and mixtures of at least two of the afore-mentioned therapeutic agents.

The growth agents may be selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleucin-1B (IL-1B), interleucin-8 (IL-8), nerve growth factor (NGF), and mixtures of at least two of the afore-mentioned growth agents.

In a further embodiment of the invention, the combination is in the form of a medical, in particular surgical, kit, wherein the collagen and/or gelatine material and the cyanoacrylate component are spatially separated from each other.

In a further embodiment of the invention, the combination is in the form of a medical, in particular surgical, device, wherein the collagen and/or gelatine material comprises or is equipped, in particular soaked, with the cyanoacrylate component.

According to a second aspect, the invention refers to a medical, in particular surgical, kit, in particular for treating and/or preventing tissue leakage. The tissue leakage may be selected from the group consisting of skin leakage, liver leakage, spleen leakage, pancreas leakage, kidney leakage, lung leakage, adrenal gland leakage, stomach leakage and intestines leakage such as small intestines leakage.

The medical kit comprises a combination comprising
- a collagen and/or gelatine material and
- a cyanoacrylate component,
wherein the collagen and/or gelatine material and cyanoacrylate component are spatially separated from each other.

In addition, the medical kit may comprise at least one further component, in particular instructions for use.

With respect to further features and advantages of the medical kit, in particular with respect to the collagen and/or gelatine material and cyanoacrylate component, reference is made in its entirety to the first aspect of the present invention. The features and advantages described under the first aspect of the present invention, in particular with respect to the collagen and/or gelatine material and cyanoacrylate component, do apply, mutatis mutandis, with respect to the medical kit according to the second aspect of the invention.

According to a third aspect, the invention refers to a medical, in particular surgical, device, in particular for treating and/or preventing tissue leakage. The tissue leakage is in particular selected from the group consisting of skin leakage, liver leakage, spleen leakage, pancreas leakage, kidney leakage, lung leakage, adrenal gland leakage, stomach leakage and intestines leakage such as small intestines leakage.

The medical device comprises a combination comprising
- a collagen and/or gelatine material and
- a cyanoacrylate component,
wherein the collagen and/or gelatine material comprises the cyanoacrylate component and/or is equipped, in particular soaked, with the cyanoacrylate component.

With respect to further features and advantages of the collagen and/or gelatine material and cyanoacrylate component, reference is made in its entirety to the first aspect of the invention. The features and advantages described under the first aspect of the invention, in particular with respect to the collagen and/or gelatine material and cyanoacrylate component, do also apply, mutatis mutandis, with respect to the medical device according to the third aspect of the invention.

According to a fourth aspect, the invention refers to a combination for use in the treatment and/or prevention of tissue leakage. The tissue leakage is in particular selected from the group consisting of skin leakage, liver leakage, spleen leakage, pancreas leakage, kidney leakage, lung leakage, adrenal gland leakage, stomach leakage and intestines leakage such as small intestines leakage.

The combination comprises
- a collagen and/or gelatine material and
- a cyanoacrylate component.

With respect to further features and advantages of the combination, in particular with respect to the collagen and/or gelatine material and cyanoacrylate component, reference is made in its entirety to the first aspect of the invention. The features and advantages described under the first aspect of the invention, in particular with respect to the collagen and/or gelatine material and cyanoacrylate component, do also apply, mutatis mutandis, with respect to the combination according to the fourth aspect of the invention.

According to a fifth aspect, the invention refers to a medical, in particular surgical, kit for use in the treatment and/or prevention of tissue leakage. The tissue leakage is in particular selected from the group consisting of skin leakage, liver leakage, spleen leakage, pancreas leakage, kidney leakage, lung leakage, adrenal gland leakage, stomach leakage and intestines leakage.

The medical kit comprises a combination comprising
- a collagen and/or gelatine material and
- a cyanoacrylate component,
wherein the collagen and/or gelatine material and cyanoacrylate component are spatially separated from each other.

With respect to further features and advantages of the medical kit, in particular the collagen and/or gelatine material and cyanoacrylate component, reference is made in its entirety to the first aspect of the invention. The features and advantages described under the first aspect of the invention, in particular with respect to the collagen and/or gelatine material and cyanoacrylate component, do also apply, mutatis mutandis, with respect to the medical kit according to the fifth aspect of the invention.

According to a sixth aspect, the invention refers to a medical, in particular surgical, device for use in the treatment and/or prevention of tissue leakage. The tissue leakage is in particular selected from the group consisting of skin leakage, liver leakage, spleen leakage, pancreas leakage, kidney leakage, lung leakage, adrenal gland leakage, stomach leakage and intestines leakage such as small intestines leakage .

The medical device comprises a combination comprising
- a collagen and/or gelatine material and
- a cyanoacrylate component,
wherein the collagen and/or gelatine material comprises the cyanoacrylate component and/or is equipped, in particular soaked, with the cyanoacrylate component.

With respect to further features and advantages of the medical device, in particular collagen and/or gelatine material and cyanoacrylate component, reference is made in its entirety to the first aspect of the invention. The features and advantages described under the first aspect of the invention, in particular with respect to the collagen and/or gelatine material and cyanoacrylate component, do also apply, mutatis mutandis, with respect to the device according to the sixth aspect of the invention.

According to a seventh aspect, the invention refers to a method for treating and/or preventing tissue leakage in a subject comprising the steps of
- equipping, in particular soaking, a collagen and/or gelatine material with a cyanoacrylate component and
- applying the collagen and/or gelatine material equipped with the cyanoacrylate component to the tissue leakage.

The subject is preferably a human subject. Alternatively, the subject may be an animal subject such as a horse, a cattle, a pig, a sheep, a goat, a dog, a cat or a rodent.

The tissue leakage is in particular selected from the group consisting of skin leakage, liver leakage, spleen leakage, pancreas leakage, kidney leakage, lung leakage, adrenal gland leakage, stomach leakage and intestines leakage such as small intestines leakage.

Further features and advantages of the invention will become clear from the following description of preferred embodiments in form of examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### Example section

Several in vitro testings were performed evaluating the inventive concept using completely different surfaces like meat, a rubber tube and sheep intestines. Using the rubber tube and sheep intestines filled with a colored solution allow the observation of the patch impermeability in function of time. Using meat as support allows the simulation of patch adherence on a moist surface.

A pilot trial was performed using a published animal model with pigs to confirm the feasibility of the concept. An intestinal anastomosis was created using a stapler, in a second step a defect in the anastomotic line of 2 cm was created simulating an anastomotic dehiscence.

A collagen membrane was soaked in Histoacryl (n-butyl 2-cyanoacrylate monomers) for approximately 30 seconds (every site, in total approximately 60 seconds) in a petri dish and applied on the defect.

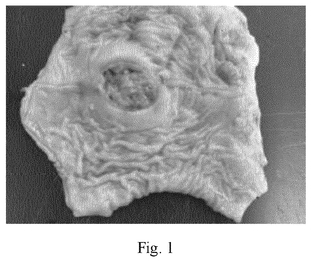

The combined medical device should also help to perform physiological remodeling of the tissue at the damaged site. It allows fibroblast migration, followed by subsequent native collagen deposition. After a follow-up of 7 days euthanasia took place, confirming the partial healing of the intestinal defect. The histological tissue examination revealed that tissue granulation took place during the healing phase (see fig. 1).

## Claims

1. A combination for treating and/or preventing tissue leakage, comprising
- a collagen and/or gelatine material and
- a cyanoacrylate component.

2. The combination of claim 1, **characterized in that** the collagen and/or gelatine material is in the form of a two-dimensional surface structure, in particular flat two-dimensional surface structure.

3. The combination of claim 1 or 2, **characterized in that** the collagen and/or gelatine material is in the form of a patch, fabric, non-woven, membrane, sponge or a combination of at least two of the aforementioned forms.

4. The combination according to any of the preceding claims, **characterized in that** the collagen and/or gelatine material is impermeable to cells.

5. The combination according to any of the claims 1 to 3, **characterized in that** the collagen and/or gelatine material is permeable to cells.

6. The combination according to any of the preceding claims, **characterized in that** the collagen and/or gelatine material comprises collagen selected from the group consisting of collagen type I, collagen type II, collagen type III; collagen type IV, collagen type V, recombinant collagen and mixtures of at least two of the aforementioned collagen types.

7. The combination according to any of the preceding claims, **characterized in that** the collagen and/or gelatine material comprises a biological tissue and/or an extracellular matrix (ECM), in particular selected from the group consisting of pericardium, pericardium fibrosum, pericardium serosum, epicardium, squamous epithelium, tunica serosa, muscle such as myocardium, peritoneum, small intestinal submucosa, gastric submucosa, urinary bladder submucosa, uterine submucosa, serosa dermis, and mixtures of at least two of the aforementioned biological tissues and extracellular matrices, respectively.

8. The combination according to any of the preceding claims, **characterized in that** the collagen and/or gelatine material is of xenogenic, in particular bovine, porcine, equine, ovine, or marine origin.

9. The combination according to any of the preceding claims, **characterized in that** the collagen and/or gelatine material is in the form of an implant, in particular surgical implant.

10. The combination according to any of the preceding claims, **characterized in that** the cyanoacrylate component includes cyanoacrylate monomers represented by the formula I
CH₂=CH(CN)-COOR (formula I)
wherein R is an alkyl, alkoxyalkyl, cycloalkyl, alkenyl, aralkyl, aryl, allyl or haloalkyl group.

11. The combination according to any of the preceding claims, **characterized in that** the cyanoacrylate component includes alkyl 2-cyanoacrylate monomers, in particular selected from the group consisting of methyl 2-cyanoacrylate monomers, ethyl 2-cyanoacrylate monomers, n-propyl 2-cyanoacrylate monomers, isopropyl 2-cyanoacrylate monomers, n-butyl 2-cyanoacrylate monomers, isobutyl 2-cyanoacrylate monomers such as, for example, 1-butyl 2-cyanoacrylate monomers and/or 2-butyl 2-cyanoacrylate monomers, n-pentyl 2-cyanoacrylate monomers, isopentyl 2-cyanoacrylate monomers such as, for example, 1-pentyl 2-cyanoacrylate monomers, 2-pentyl 2-cyanoacrylate monomers and/or 3-pentyl 2-cyanoacrylate monomers, cyclopentyl 2-cyanoacrylate monomers, n-hexyl 2-cyanoacrylate monomers, isohexyl 2-cyanoacrylate monomers such as, for example, 1-hexyl 2-cyanoacrylate monomers, 2-hexyl 2-cyanoacrylate monomers, 3-hexyl 2-cyanoacrylate monomers and/or 4-hexyl 2-cyanoacrylate monomers, cyclohexyl 2-cyanoacrylate monomers, n-heptyl 2-cyanoacrylate monomers, isoheptyl 2-cyanoacrylate monomers such as, for example, 1-heptyl 2-cyanoacrylate monomers, 2-heptyl 2-cyanoacrylate monomers, 3-heptyl 2-cyanoacrylate monomers and/or 4-heptyl 2-cyanoacrylate monomers, n-octyl 2-cyanoacrylate monomers, isooctyl 2-cyanoacrylate monomers such as, for example, 1-octyl 2-cyanoacrylate monomers, 2-octyl 2-cyanoacrylate monomers, 3-octyl 2-cyanoacrylate monomers and/or 4-octyl 2-cyanoacrylate monomers, n-nonyl 2-cyanoacrylate monomers, isononyl 2-cyanoacrylate monomers, n-decyl 2-cyanoacrylate monomers, isodecyl 2-cyanoacrylate monomers, n-undecyl 2-cyanoacrylate monomers, isoundecyl 2-cyanoacrylate monomers, n-dodecyl 2-cyanoacrylate monomers, isododecyl 2-cyanoacrylate monomers and mixtures of at least two of the aforementioned alkyl 2-cyanoacrylate monomers.

12. The combination according to any of the preceding claims, **characterized in that** the combination is in the form of a medical, in particular surgical, kit, wherein the collagen and/or gelatine material and the cyanoacrylate component are spatially separated from each other.

13. The combination according to any of the claims 1 to 11, **characterized in that** the combination is in the form of a medical, in particular surgical, device, wherein the collagen and/gelatine material is equipped with the cyanoacrylate component.

14. Medical kit for treating and/or preventing tissue leakage, comprising a combination according to any of the claims 1 to 11.

15. Medical device for treating and/or preventing tissue leakage, comprising a combination according to any of the claims 1 to 11.
